(19) Europäisches Patentamt
European Patent Office
Office européen des brevets

(11) EP 1 162 204 B1

(12) EUROPÄISCHE PATENTSCHRIFT

(45) Veröffentlichungstag und Bekanntmachung des Hinweises auf die Patenterteilung:
**20.07.2005 Patentblatt 2005/29**

(51) Int Cl.[7]: **C07F 9/28**, H01M 10/40, C07D 233/58

(21) Anmeldenummer: **01111953.4**

(22) Anmeldetag: **21.05.2001**

# (54) Ionische Flüssigkeiten

Ionic liquids

Liquides ioniques

(84) Benannte Vertragsstaaten:
**DE FR GB**

(30) Priorität: **09.06.2000 DE 10027995**

(43) Veröffentlichungstag der Anmeldung:
**12.12.2001 Patentblatt 2001/50**

(73) Patentinhaber: **MERCK PATENT GmbH
64293 Darmstadt (DE)**

(72) Erfinder:
- **Schmidt, Michael
64342 Seeheim-Jugenheim (DE)**
- **Heider, Udo
64560 Riedstadt (DE)**
- **Geissler, Winfried
64380 Rossdorf (DE)**
- **Ignatyev, Nikolai
47058 Duisburg (DE)**
- **Hilarius, Volker
64823 Gross-Umstadt (DE)**

(56) Entgegenhaltungen:
**WO-A-98/15562** **US-A- 5 827 602**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ionische Flüssigkeiten zur Anwendung in elektrochemischen Zellen und organischen Synthesen.

Lösungsmittelfreie ionische Flüssigkeiten oder bei "Raumtemperatur geschmolzene Salze"wurden erstmals in US 2446331 beschrieben. Das Problem dieser starken Lewis-Säuren ist die Bildung giftiger Gase bei Kontakt mit Luftfeuchtigkeit.

Lange Zeit wurden Verbindungen um $AlCl_3$ und 1-Ethyl-3-methylimidazolium (EMI) chlorid untersucht. Wilkes und Zaworotko stellten 1992 in J. Chem. Soc., Chem. Commun., S. 965 neue Lösungsmittelfreie ionische Flüssigkeiten, EMI $BF_4$ und EMI $O_2CCH_3$, vor. Allerdings sind diese Verbindungen für den Einsatz als Elektrolyt in elektrochemischen Zellen ungeeignet, da die $BF_4^-$ und $CH_3C_2^-$ Anionen schon bei relativ niedrigen Potentialen oxidiert werden.

In DE 196 41 138 wird eine neue Klasse von Leitsalzen, die Lithiumfluoralkylphosphate, beschrieben. Diese Salze zeichnen sich durch eine hohe elektrochemische Stabilität und geringe Hydrolyseneigung aus (M. Schmidt et al. 10th International Meeting on Lithium Batteries, Como 2000). Bei Zyklisierungsversuchen haben diese Verbindungen besonders gute Ergebnisse gezeigt und sich als besonders stabil erwiesen.

In US 5827602 wird der Einsatz ionischerFlüssigkeiten aus der Gruppe der Pyridinium-, Pyridazinium-, Pyrimidinium-, Pyrazinium-, Imidazolium-, Pyrazolium-, Thiazolium-, Oxazolium- und Triazoliumsalze in elektrochemischen Zellen beschrieben, die Imide oder Methanide als Anionen enthalten. Diese ionischen Flüssigkeiten sind durch gute Leitfähigkeiten für diese Anwendung besonders geeignet. Der entscheidende Nachteil besteht in der teuren Synthese der Rohstoffe, insbesondere der Anionen.

Gegenstand der vorliegenden Erfindung ist es deshalb, ionische Flüssigkeiten zur Verfügung zu stellen, die einen großen Flüssigkeitsbereich, eine hohe thermische Stabilität und geringe Korrosivität sowie kostengünstiger synthetisierte Anionen aufweisen.

Die erfindungsgemäße Aufgabe wird gelöst durch ionische Flüssigkeiten der allgemeinen Formel

$$K^+A^- \qquad (I)$$

worin bedeuten:

$K^+$ ein Kation ausgewählt aus der Gruppe

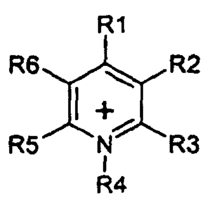

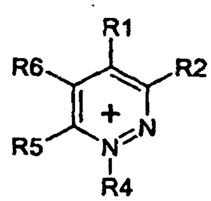

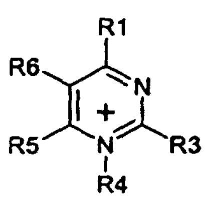

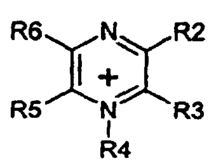

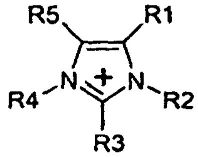

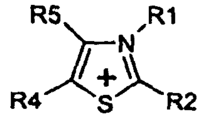

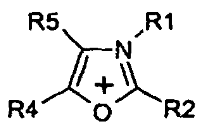 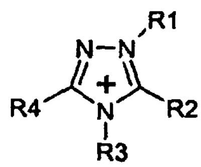

wobei $R^1$ bis $R^5$ gleich oder verschieden, gegebenenfalls durch eine Einfach- oder Doppelbindung direkt miteinander verbunden sind und jeweils einzeln oder gemeinsam folgende Bedeutung haben:

-H,

-Halogen,

-Alkylrest ($C_1$ bis $C_8$) der teilweise oder vollständig durch weitere Gruppen, vorzugsweise F, Cl, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit $1<n<6$ und $0<x\leq 2n+1$ substituiert sein kann

und

$A^-$ Tris(pentafluorethyl)trifluorphosphat oder Tris(nonafluorbutyl)trifluorphosphat bedeutet.

Diese ionischen Flüssigkeiten sind als Lösungsmittel in der organischen Synthese, aber auch für den Einsatz in elektrochemischen Zellen geeignet. Zudem sind die ionischen Flüssigkeiten für den Einsatz in der Katalyse von chemischen Reaktionen geeignet. Außerdem können sie als inertes Lösungsmittel für hochreaktive Chemikalien verwendet werden. Ein weiteres Gebiet ist die Verwendung als hydraulische Flüssigkeit.

Es wurde gefunden, daß die erfindungsgemäßen Verbindungen durch den Einsatz perfluorierter Alkylketten hydrophob sind, wobei längerkettige perfluorierte Alkylketten bevorzugt sind. Durch die wasserfreie Synthese wird zudem der unerwünschte Eintrag von Wasser in das System minimiert.

Überraschend wurde gefunden, daß die ionischen Flüssigkeiten den Aluminium-Stromkollektor, der üblicherweise in elektrochemischen Zellen verwendet wird, nicht korrodieren, sondern sogar passivieren. Damit kann die Zyklenstabilität erhöht werden. Außerdem konnte eine verbesserte thermische Stabilität des Systems durch den Einsatz ionischer Flüssigkeiten festgestellt werden.

Es wurde gefunden, daß durch den Zusatz von Lösungsmitteln mit niedriger Viskosität die Leitfähigkeit verbessert werden kann. Eine niedrige Viskosität mit hoher Leitfähigkeit ist für den Einsatz in elektrochemischen Zellen Voraussetzung. Die erfindungsgemäßen Verbindungen weisen einen großen Flüssigkeitsbereich auf, so daß sie für diese Anwendungen besonders geeignet sind.

Für die Anwendung in Doppelschichtkondensatoren, beispielsweise in Superkondensatoren, ist eine hohe Leitfähigkeit Voraussetzung. Die erfindungsgemäßen Verbindungen erfüllen dieses Kriterium und können daher allein oder in Mischungen mit anderen Lösungsmitteln oder Leitsalzen eingesetzt werden. Die ionischen Flüssigkeiten wie in Anspruch 1 definiert, können in Mischungen mit aprotischen Lösungsmitteln verwendet werden. Geeignet sind Lösungsmittel ausgewählt aus der Gruppe organischer Carbonate (z.B. Ethylencarbonat, Propylencarbonat und deren Derivate, Butylencarbonat, Dimethylcarbonat, Diethylcarbonat, Ethylmethylcarbonat usw.),organischer Carbonsäureester (z.B. γ-Butyrolacton, Methylformiat, Methylacetat, Ethylacetat, Ethylpropionat, Methylpropionat, Methylbutyrat, Ethylbutyrat usw.), organischer Carbonsäureamide (z.B. Dimethylformamid, Methylformamid, Formamid usw.), organischer Ether (z.B. 1,2-Dimethoxyethan, Tetrahydrofuran, 2-Methyltetrahydrofuran, Tetrahydrofuranderivate, 1,3-Dioxolan, Dioxan, Dioxolanderivate usw.) oder andere aprotische Lösungsmittel (z.B. Acetonitril, Sulfolan, Dimethylsulfoxid, Nitromethan, Phosphorsäuretriester, Trimethoxymethan, 3-Methyl-2-oxazolidinon usw.). Lösungsmittelgemische, wie z.B. Ethylencarbonat/Dimethylcarbonat (EC/DMC) können ebenfalls verwendet werden.

Die erfindungsgemäßen Verbindungen können in gängigen Elektrolyten eingesetzt werden. Sie können in Mischungen zwischen 1 und 99% enthalten sein. Geeignet sind z.B. Elektrolyte mit Leitsalzen ausgewählt aus der Gruppe $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen.

Die Elektrolyte können auch organische Isocyanate (DE 199 44 603) zur Herabsetzung des Wassergehaltes enthalten.

Auch Komplexsalze der allgemeinen Formel (DE 199 51 804)

$$M^{x+}[EZ]^{y-}_{x/y}$$

worin bedeuten:

x,y 1,2,3

$M^{x+}$ ein Metallion

E eine Lewis-Säure, ausgewählt aus der Gruppe

$BR^1R^2R^3$, $AlR^1R^2R^3$, $PR^1R^2R^3R^4R^5$, $AsR^1R^2R^3R^4R^5$, $VR^1R^2R^3R^4R^5$,
$R^1$ bis $R^5$ gleich oder verschieden, gegebenenfalls durch eine Einfach- oder Doppelbildung direkt miteinander verbunden sind, jeweils einzeln oder gemeinsam die Bedeutung
eines Halogens (F, Cl, Br),
eines Alkyl- oder Alkoxyrestes ($C_1$ bis $C_8$) der teilweise oder vollständig durch F, Cl, Br substituiert sein kann,
eines, gegebenenfalls über Sauerstoff gebundenen aromatischen Rings aus der Gruppe Phenyl, Naphthyl, Anthracenyl oder Phenanthrenyl, der unsubstituiert oder ein- bis mehrfach durch Alkyl ($C_1$ bis $C_8$) oder F, Cl, Br substituiert sein kann
eines, gegebenenfalls über Sauerstoff gebundenen aromatischen heterocyclischen Rings aus der Gruppe Pyridyl, Pyrazyl oder Pyrimidyl, der unsubstituiert oder ein- bis mehrfach durch Alkyl ($C_1$ bis $C_8$) oder F, Cl, Br substituiert sein kann, haben können und
Z $OR^6$, $NR^6R^7$, $CR^6R^7R^8$, $OSO_2R^6$, $N(SO_2R^6)(SO_2R^7)$,
$C(SO_2R^6)(SO_2R^7)(SO_2R^8)$, $OCOR^6$, wobei
$R^6$ bis $R^8$ gleich oder verschieden sind, gegebenenfalls durch eine Einfach- oder Doppelbindung direkt miteinander verbunden sind, jeweils einzeln oder gemeinsam die Bedeutung
eines Wasserstoffs oder die Bedeutung wie $R^1$ bis $R^5$ haben, hergestellt durch Umsetzung von einem entsprechenden Bor- oder Phosphor-Lewis-Säure-Solvenz-Adukt mit einem Lithium- oder Tetraalkylammonium-Imid, -Methanid oder -Triflat, können enthalten sein.

Auch Boratsalze (DE 199 59 722) der allgemeinen Formel

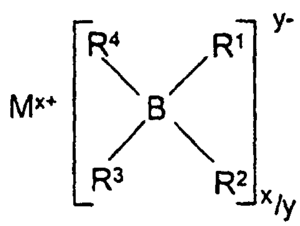

worin bedeuten:

M ein Metallion oder Tetraalkylammoniumion

x,y 1, 2 oder 3

$R^1$ bis $R^4$ gleich oder verschieden, gegebenenfalls durch eine Einfach- oder Doppelbindung direkt miteinander verbundener Alkoxy- oder Carboxyreste ($C_1$-$C_8$) können enthalten sein. Hergestellt werden diese Boratsalze durch Umsetzung von Lithiumtetraalkoholatborat oder einem 1:1 Gemisch aus Lithiumalkoholat mit einem Borsäureester in einem aprotischen Lösungsmittel mit einer geeigneten Hydroxyl- oder Carboxylverbindung im Verhältnis 2:1 oder 4:1.

Auch Additive wie Silanverbindungen der allgemeinen Formel

$$SiR^1R^2R^3R^4$$

mit $R^1$ bis $R^4$ H
$C_yF_{2y+1-z}H_z$
$OC_yF_{2y+1-z}H_z$
$OC(O)C_yF_{2y+1-z}H_z$
$OSO_2C_yF_{2y+1-z}H_z$
und $1 \leq x < 6$
$1 \leq y \leq 8$ und

$0 \leq z \leq 2y+1$
und
$R^1$-$R^4$ gleich oder verschieden
mit der Bedeutung eines aromatischen Rings aus der Gruppe Phenyl, Naphthyl der unsubstituiert oder ein- oder mehrfach durch F, $C_yF_{2y+1-z}H_z$ oder $OC_yF_{2y+1-z}H_z$, $OC(O)C_yF_{2y+1-z}H_z$, $OSO_2C_yF_{2y+1-z}H_z$, $N(C_nF_{2n+1-z}H_z)_2$ substituiert sein kann, oder
mit der Bedeutung eines heterocyclischen aromatischen Rings aus der Gruppe Pyridyl, Pyrazyl oder Pyrimidyl, die jeweils ein- oder mehrfach mit F, $C_yF_{2y+1-z}H_z$ oder $OC_yF_{2y+1-z}H_z$, $OC(O)C_yF_{2y+1-z}H_z$, $OSO_2C_yF_{2y+1-z}H_z$, $N(C_nF_{2n+1-z}H_z)_2$ substituiert sein können (DE 100 276 26), können enthalten sein.

Die erfindungsgemäßen Verbindungen können auch in Elektrolyte eingesetzt werden, die Lithiumfluoralkylphosphate der folgenden Formel enthalten,

$$Li^+[PF_x(C_yF_{2y+1-z}H_z)_{6-x}]^-$$

worin
$1 \leq x \leq 5$
$3 \leq y \leq 8$
$0 \leq z \leq 2y + 1$
bedeuten und die Liganden $(C_yF_{2y+1-z}H_z)$ gleich oder verschieden sein können, wobei die Verbindungen der allgemeinen Formel,

$$Li^+[PF_a(CH_bF_c(CF_3)_d)_e]^-$$

in der a eine ganze Zahl von 2 bis 5, b = 0 oder 1, c = 0 oder 1, d = 2 und
e eine ganze Zahl von 1 bis 4 bedeuten, mit den Bedingungen, daß b und c nicht gleichzeitig jeweils = 0 bedeuten und die Summe aus a + e gleich 6 ist und die Liganden $(CH_bF_c(CF_3)_d)$ gleich oder verschieden sein können, ausgenommen sind (DE 100 089 55). Das Verfahren zur Herstellung von Lithiumfluoralkylphosphaten ist dadurch gekennzeichnet, daß wenigstens eine Verbindung der allgemeinen Formel

$$H_mP(C_nH_{2n+1})_{3-m},$$

$$OP(C_nH_{2n+1})_3,$$

$$Cl_mP(C_nH_{2n+1})_{3-m},$$

$$F_mP(C_nH_{2n+1})_{3-m},$$

$$Cl_oP(C_nH_{2n+1})_{5-o},$$

$$F_oP(C_nH_{2n+1})_{5-o},$$

in denen jeweils
$0 < m < 2$, $1 < n < 8$ und $0 < o < 4$ bedeutet
durch Elektrolyse in Fluorwasserstoff fluoriert wird, das so erhaltene Gemisch der Fluorierungsprodukte durch Extraktion, Phasentrennung und/oder Destillation aufgetrennt wird, und das so erhaltene fluorierte Alkylphosphoran in einem aprotischen Lösungsmittel oder Lösungsmittelgemisch unter Feuchtigkeitsausschluß mit Lithiumfluorid umgesetzt wird, und das so erhaltene Salz nach den üblichen Methoden gereinigt und isoliert wird.

Die erfindungsgemäßen Verbindungen können auch in Elektrolyten eingesetzt werden, die Salze der Formel

$$Li[P(OR^1)_a(OR^2)_b(OR^3)_c(OR^4)_dF_e]$$

worin $0 < a+b+c+d \leq 5$ und $a+b+c+d+e=6$ gilt, und $R^1$ bis $R^4$ unabhängig voneinander Alkyl-, Aryl- oder Heteroarylreste sind, wobei mindestens zwei von $R^1$ bis $R^4$ durch eine Einfach- oder Doppelbindung direkt miteinander verbunden sein können, enthalten (DE 100 16 801). Dargestellt werden die Verbindungen durch Umsetzung von Phosphor (V) -Verbindungen der allgemeinen Formel

$$P(OR^1)_a(OR^2)_b(OR^3)_c(OR^4)_dF_e$$

worin $0 < a+b+c+d \leq 5$ und $a+b+c+d+e=5$ gilt, und $R^1$ bis $R^4$ die oben angegebenen Bedeutungen haben mit Lithiumfluorid in Gegenwart eines organischen Lösungsmittels.

Auch Ionische Flüssigkeiten der allgemeinen Formel

$$K^+A^-$$

worin bedeuten:

$K^+$ ein Kation ausgewählt aus der Gruppe

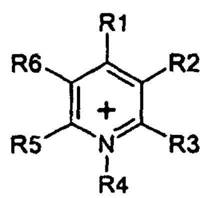
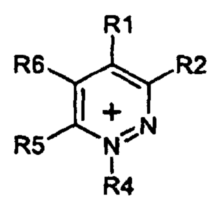

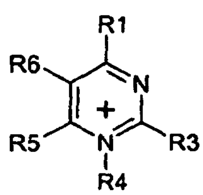
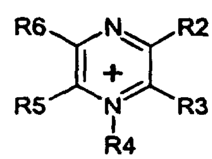

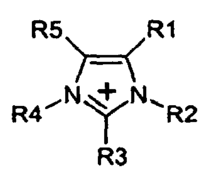
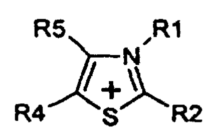

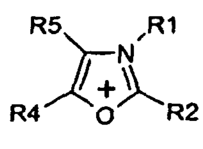
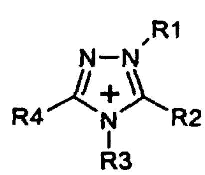

wobei $R^1$ bis $R^5$ gleich oder verschieden, gegebenenfalls durch eine Einfach- oder Doppelbindung direkt miteinander verbunden sind und jeweils einzeln oder gemeinsam folgende Bedeutung haben:

- H,
- Halogen,
- Alkylrest ($C_1$ bis $C_8$) der teilweise oder vollständig durch weitere Gruppen, vorzugsweise F, Cl, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1<n<6 und 0<x≤2n+1 substituiert sein kann

und

$A^-$ ein Anion ausgewählt aus der Gruppe

$$[B(OR^1)_n(OR^2)_m(OR^3)_o(OR^4)_p]^-$$

mit 0≤n, m, o, p≤4 und m+n+o+p=4 wobei $R^1$ bis $R^4$ verschieden oder paarweise gleich sind, gegebenenfalls durch eine Einfach- oder Doppelbildung direkt miteinander verbunden sind, jeweils einzeln oder gemeinsam die Bedeutung eines aromatischen Rings aus der Gruppe Phenyl, Naphthyl, Anthracenyl oder Phenanthrenyl, der unsubstituiert oder ein- oder mehrfach durch $C_nF_{(2n+1-x)}H_x$ mit 1 <n<6 und 0<x≤13 oder Halogen (F, Cl, Br) substituiert sein kann, besitzen, die Bedeutung eines aromatischen heterocyclischen Rings aus der Gruppe Pyridyl, Pyrazyl oder Pyrimidyl, der unsubstituiert oder ein- oder mehrfach durch $C_nF_{(2n+1-x)}H_x$ mit 1<n<6 und 0<x≤13 oder Halogen oder Halogen (F, Cl, Br) substituiert sein kann, besitzen, die Bedeutung eines Alkylrests ($C_1$ bis $C_8$) der teilweise oder vollständig durch weitere Gruppen, vorzugsweise F, Cl, , $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1<n<6 und 0<x≤13 substituiert sein kann, besitzen, beziehungsweise $OR^1$ bis $OR^4$ einzeln oder gemeinsam die Bedeutung eines aromatischen oder aliphatischen Carboxyl-, Dicarboxyl-, Oxysulfonyl- oder Oxycarboxylrests, der teilweise oder vollständig durch weitere Gruppen, vorzugsweise F, Cl, , $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1<n<6 und 0<x≤13 substituiert sein kann, besitzen (DE 100 265 65), können im Elektrolyten enthalten sein. Auch lonische Flüssigkeiten $K^+A^-$ mit $K^+$ definiert wie oben und

Die erfindungsgemäßen Verbindungen können auch in Elektrolyten enthalten sein mit Verbindungen folgender Formel:

$$NR^1R^2R^3$$

worin

$R^1$ und scher $R^2H$, $C_yF_{2y+1-z}H_z$ oder $(C_nF_{2n-m}H_m)X$, wobei X ein aromatischer oder heterozyklischer Rest ist, bedeuten und

$R^3$ $(C_nF_{2n-m}H_m)Y$, wobei Y ein heterozyklischer Rest ist, oder

$(C_oF_{2o-p}H_p)Z$, wobei Z ein aromatischer Rest ist, bedeutet, und wobei n, m, o, p, y und z die folgenden Bedingungen erfüllen:
$0 \le n \le 6$,
$0 \le m \le 2n$,
$2 \le o \le 6$,
$0 \le p \le 2o$,
$1 \le y \le 8$ und
$0 \le z \le 2y+1$, zur Verringerung des Säuregehalts in aprotischen Elektrolytsystemen in elektrochemischen Zellen.

Auch Fluoralkylphosphate der allgemeinen Formel

$$M^{n+}[PF_x(C_yF_{2y+1-z}H_z)_{6-x}]_n^-$$

worin
$1 \leq x \leq 6$
$1 \leq y \leq 8$
$0 \leq z \leq 2y + 1$
$1 \leq n \leq 3$ und
$M^{n+}$ die Bedeutung eines ein- bis dreiwertigen Kations besitzt, insbesondere:

$NR^1R^2R^3R^4$,

$PR^1R^2R^3R^4$,

$P(NR^1R^2)_kR^3{}_mR^4{}_{4-k-m}$ (mit k=1-4, m=0-3 und k+m≤4),

$C(NR^1R^2)(NR^3R^4)(NR^5R^6)$,

$C(Aryl)_3$, Rb oder Tropylium

mit $R^1$ bis $R^8$ H, Alkyl und Aryl ($C_1$-$C_8$) die teilweise durch F, Cl oder Br substituiert sein können,

wobei $M^{n+} = Li^+$, $Na^+$, $Cs^+$, $K^+$ und $Ag^+$ ausgenommen sind, können enthalten sein. Diese Fluoralkylphosphate sind erhältlich indem Phosphorane mit einem Fluorid oder Metallfluoralkylphosphate mit einem Fluorid oder Chlorid in organischen aprotischen Lösungsmitteln umgesetzt werden (DE 100 388 58).

Der Elektrolyt kann auch ein Gemisch enthalten, daß

a) wenigstens ein Lithiumfluoroalkylphosphat-Salz der allgemeinen Formel

$$Li^+ [PF_x(C_yF_{2y+1-z}H_z)_{6-x}]^-$$

worin
$1 \leq x \leq 5$
$1 \leq y \leq 8$ und
$0 \leq z \leq 2y + 1$ bedeuten und die Liganden $(C_yF_{2y+1-z}H_z)$ jeweils gleich oder verschieden sind und
b) wenigstens ein Polymer (DE 100 58 264) enthält.

Im Elektrolyten können auch Tetrakisfluoroalkylborat-Salze der allgemeinen Formel

$$M^{n+} ([BR_4]^-)_n$$

worin
$M^{n+}$ ein einwertiges, zweiwertiges oder dreiwertiges Kation ist,
die Liganden R jeweils gleich sind und für $(C_xF_{2x+1})$ mit $1 \leq x \leq 8$ stehen
und n = 1, 2 oder 3 ist (DE 100 558 11) enthalten sein. Das Verfahren zur Herstellung von Tetrakisfluoroalkylborat-Salzen ist dadurch gekennzeichnet, daß wenigstens eine Verbindung der allgemeinen Formel $M^{n+} ([B(CN)_4]^-)_n$ worin $M^{n+}$ und n die oben angegebene Bedeutung haben, durch Umsetzung mit wenigstens einem Fluorierungsmittel in wenigstens einem Lösungsmittel fluoriert und die so erhaltene fluorierte Verbindung nach den üblichen Methoden gereinigt und isoliert wird.

Der Elektrolyt kann auch Boratsalze der allgemeinen Formel

$$M^{n+} [BF_x(C_yF_{2y+1-z}H_z)_{4-x}]_n^-$$

enthalten, worin bedeuten:

$$1<x<3,\ 1 \leq y \leq 8 \text{ und } 0 \leq z \leq 2y+1$$

und

M ein- bis dreiwertiges Kation ($1 \leq n \leq 3$), außer Kalium und Barium, insbesondere:

Li,

$NR^1R^2R^3R^4$, $PR^5R^6R^7R^8$, $P(NR^5R^6)_kR^7_mR^8_{4-k-m}$ (mit k=1-4, m=0-3 und $k+m \leq 4$) oder

$C(NR^5R^6)(NR^7R^8)(NR^9R^{10})$, wobei

$R^1$ bis $R^4$ $C_yF_{2y+1-z}H_z$ und

$R^5$ bis $R^{10}$ H oder $C_yF_{2y+1-z}H_z$ oder ein aromatisches heterozyklisches Kation, insbesondere Stickstoff- und/oder Sauerstoff- und/oder Schwefelhaltige aromatische heterozyklische Kationen (DE 101 031 89). Das Verfahren zur Herstellung dieser Verbindungen ist dadurch gekennzeichnet, daß

a) $BF_3$-Lösungsmittel-Komplexe 1:1 mit Alkyllithium unter Kühlung umgesetzt werden, nach langsamer Erwärmung das Lösungsmittel zu einem Großteil entfernt und anschließend der Feststoff abfiltriert und mit einem geeigneten Lösungsmittel gewaschen wird, oder

b) Lithiumsalze in einem geeigneten Lösungsmittel 1:1 mit $B(CF_3)F_3$-salzen umgesetzt werden, bei erhöhter Temperatur gerührt und nach Entfernen des Lösungsmittels das Reaktionsgemisch mit aprotischen nichtwäßrigen Lösungsmitteln, vorzugsweise mit Lösungsmitteln die in elektrochemischen Zellen verwendet werden, versetzt und getrocknet wird, oder

c) $B(CF_3)F_3$-salze 1:1 bis 1:1,5 mit Lithiumsalzen in Wasser bei erhöhter Temperatur umgesetzt und 0,5 bis 2 Stunden am Siedepunkt erhitzt werden, das Wasser entfernt und das Reaktionsgemisch mit aprotischen nichtwäßrigen Lösungsmitteln, vorzugsweise mit Lösungsmitteln die in elektrochemischen Zellen verwendet werden, versetzt getrocknet wird.

Im Elektrolyten können auch Fluoroalkylphosphat-Salze der allgemeinen Formel

$$M^{n+} ([PF_x(C_yF_{2y+1-z}H_z)_{6-x}]^-)_n$$

enthalten sein, worin
$M^{n+}$ ein einwertiges, zweiwertiges oder dreiwertiges Kation ist,
$1 \leq x \leq 5$,
$1 \leq y \leq 8$ und
$0 \leq z \leq 2y + 1$ sind, n = 1, 2 oder 3 bedeuten und die Liganden ($C_yF_{2y+1-z}H_z$) jeweils gleich oder verschieden sind, wobei die Fluoroalkylphosphat-Salze, in denen $M^{n+}$ ein Lithium-Kation ist, sowie die Salze

$$M^+([PF_4(CF_3)_2]^-) \text{ mit } M^+ = Cs^+, Ag^+ \text{ oder } K^+,$$

$$M^+([PF_4(C_2F_5)_2]^-) \text{ mit } M^+ = Cs^+,$$

$$M^+([PF_3(C_2F_5)_3]^-) \text{ mit } M^+ = Cs^+, K^+, Na^+ \text{ oder para-}Cl(C_6H_4)N_2^+,$$

$$M^+([PF_3(C_3F_7)_3]^-) \text{ mit } M^+ = Cs^+, K^+, Na^+, \text{para-}Cl(C_6H_4)N_2^+$$

oder para-$O_2N(C_6H_4)N_2^+$, ausgenommen sind (DE 100 558 12). Das Verfahren zur Herstellung dieser Fluoroalkylphosphat-Salze ist dadurch gekennzeichnet, daß wenigstens eine Verbindung der allgemeinen Formel

$$H_rP(C_sH_{2s+1})_{3-r},$$

$$OP(C_sH_{2s+1})_3,$$

$$Cl_rP(C_sH_{2s+1})_{3-r},$$

$$F_rP(C_sH_{2s+1})_{3-r},$$

$$Cl_tP(C_sH_{2s+1})_{5-t}$$

und/oder

$$F_tP(C_sH_{2s+1})_{5-t},$$

in denen jeweils
$0 \leq r \leq 2$
$3 \leq s \leq 8$ und
$0 \leq t \leq 4$ bedeuten, durch Elektrolyse in Fluorwasserstoff fluoriert wird, das so erhaltene Gemisch der Fluorierungsprodukte aufgetrennt wird und das so erhaltene fluorierte Alkylphosphoran in einem aprotischen Lösungsmittel oder Lösungsmittelgemisch unter Feuchtigkeitsausschluß mit einer Verbindung der allgemeinen Formel $M^{n+}(F^-)_n$ worin $M^{n+}$ und n die oben angegebene Bedeutung haben, umgesetzt wird, und das so erhaltene Fluoroalkylphosphat-Salz nach den üblichen Methoden gereinigt und isoliert wird.

Die erfindungsgemäßen Verbindungen können in Elektrolyten enthalten sein, die Fluoralkylphosphat-Salze (DE 10109032) der Formel

$$(M^{a+})_b[(C_nF_{2n+1-m}H_m)_yPF_{5-y}(CR_1R_2)_xPF_{5-y}(C_nF_{2n+1-m}H_m)_y]^{(2-)}\ (a*b/2)$$

enthalten, worin
$M^{a+}$ ein einwertiges, zweiwertiges oder dreiwertiges Kation,
a = 1, 2 oder 3, b = 2 für a = 1, b = 2 für a = 3, b = 1 für a = 2
und jeweils
$1 \leq n \leq 8$,
$0 \leq m \leq 2$ für n = 1 oder 2,
$0 \leq m \leq 4$ für $3 \leq n \leq 8$,
$1 \leq x \leq 12$,
$0 \leq y \leq 2$,
wobei $R_1$ und $R_2$ jeweils identisch oder unterschiedlich sind und ausgewählt sind aus der Gruppe der Fluor-, Wasserstoff-, Alkyl-, Fluoralkyl und Perfluoralkylsubstituenten und,
wobei jeweils die Substituenten $(C_nF_{2n+1-m}H_m)$ identisch oder unterschiedlich sind. Hergestellt werden diese Verbindungen, indem wenigstens ein Fluoro-$\alpha,\omega$-bis(alkylfluorophosphorano)alkan mit wenigstens einem Fluorid-Salz der allgemeinen Formel $(M^{a+})\ [F^-]_a$ worin $(M^{a+})$ und a die oben angegebene Bedeutung hat, in Lösung zu einem Fluoralkylphosphat-Salz umgesetzt und dieses gegebenenfalls nach üblichen Methoden gereinigt und/oder isoliert wird.

Die erfindungsgemäßen Verbindungen können in Elektrolyte für elektrochemische Zellen eingesetzt werden, die Anodenmaterial, bestehend aus beschichteten Metallkernen, ausgewählt aus der Gruppe Sb, Bi, Cd, In, Pb, Ga und Zinn oder deren Legierungen, enthalten (DE 100 16 024). Das Verfahren zur Herstellung dieses Anodenmaterials ist dadurch gekennzeichnet, daß

a) eine Suspension oder ein Sol des Metall- oder Legierungskerns in Urotropin hergestellt wird,

b) die Suspension mit Kohlenwasserstoffen mit $C_5$-$C_{12}$ emulgiert werden,

c) die Emulsion auf die Metall- oder Legierungskerne aufgefällt werden und

d) durch Temperung des Systems die Metallhydroxide bzw. -oxihydroxide in das entsprechende Oxid übergeführt werden.

Die erfindungsgemäßen Verbindungen können auch in Elektrolyte für elektrochemische Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden (DE 199 22 522) beschichtet sind. Sie können auch aus Lithium-Mischoxid-Partikel bestehen, die mit einem oder mehreren Polymeren (DE 199 46 066) beschichtet sind. Ebenso können die erfindungsgemäßen Verbindungen in Systemen mit Kathoden eingesetzt werden, die aus Lithium-Mischoxid-Partikeln bestehen, die mit Alkalimetallverbindungen und Metalloxiden ein- oder mehrfach beschichtet sind (DE 100 14 884). Das Verfahren zur Herstellung dieser Materialien ist dadurch gekennzeichnet, daß die Partikel in einem organischen Lösungsmittel suspendiert werden, eine Alkalimetallsalzverbindung suspendiert in einem organischen Lösungsmittel zugegeben wird, Metalloxide gelöst in einem organischen Lösungsmittel zugegeben werden, die Suspension mit einer Hydrolyselösung versetzt wird und anschließend die beschichteten Partikel abfiltriert, getrocknet und calciniert werden. Ebenso können die erfindungsgemäßen Verbindungen in Systemen eingesetzt werden, die Anodenmaterialien mit dotiertem Zinnoxid enthalten (DE 100 257 61). Dieses Anodenmaterial wird hergestellt indem

a) eine Zinnchlorid-Lösung mit Harnstoff versetzt wird,

b) die Lösung mit Urotropin und einer geeigneten Dotierverbindung versetzt wird,

c) das so erhaltene Sol in Petrolether emulgiert wird,

d) das erhaltene Gel gewaschen und das Lösungsmittel abgesaugt sowie

e) das Gel getrocknet und getempert wird.

Ebenso können die erfindungsgemäßen Verbindungen in Systemen eingesetzt werden, die Anodenmaterialien mit reduziertem Zinnoxid enthalten (DE 100 257 62). Dieses Anodenmaterial wird hergestellt indem

a) eine Zinnchlorid-Lösung mit Harnstoff versetzt wird,

b) die Lösung mit Urotropin versetzt wird,

c) das so erhaltene Sol in Petrolether emulgiert wird,

d) das erhaltene Gel gewaschen und das Lösungsmittel abgesaugt wird,

e) das Gel getrocknet und getempert wird und

f) das erhaltene $SnO_2$ in einem begasbaren Ofen einem reduzierendem Gasstrom ausgesetzt wird.

Nachfolgend wird ein allgemeines Beispiel der Erfindung näher erläutert.

Zur Darstellung des Anions Tris(pentafluorethyl)trifluorphosphat oder Tris(nonafluorbutyl)trifluorphosphat wird ein bekanntes Verfahren aus DE 196 411 38 verwendet.

Zur Darstellung des Kations ausgewählt aus der Gruppe

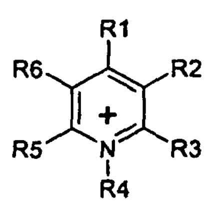 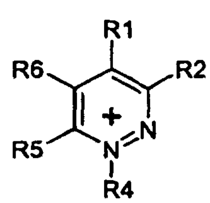

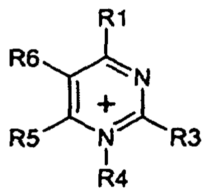
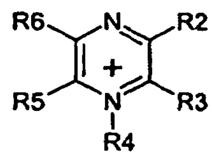

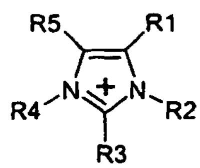
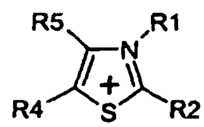

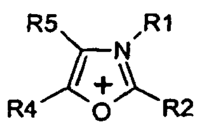
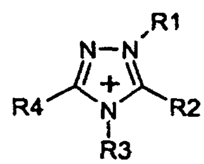

wird ein bekanntes Verfahren aus US 5827602 verwendet. Die Edukte werden in einem aprotischen organischen Lösungsmittel, bei Temperaturen im Flüssigkeitsbereich des Lösungsmittels, ca. 0,5 bis 12 Stunden, bevorzugt 1-4 Stunden, umgesetzt.

Zur Entfernung der Nebenprodukte wird auf bis zu -30°C, z.B. bei LiCl als Nebenprodukt auf -10°C bis -20°C, gekühlt und das ausfallende Nebenprodukt abfiltriert, bevorzugt vakuumfiltriert.

Das Lösungsmittel/Produkt-Gemisch kann direkt im Elektrolyten eingesetzt werden. Gegebenenfalls kann auch das Lösungsmittel abdestilliert und das erhaltene Produkt getrocknet werden, um in den angegebenen Anwendungen eingesetzt zu werden.

Die nachfolgenden Beispiele sollen die Erfindung näher erläutern, ohne sie jedoch zu beschränken.

Beispiele

Beispiel 1

Synthese von 1-Ethyl-3-methylimidazolium-tris(pentafluorethyl)trifluorphosphat

Lithium tris(pentafluorethyl)trifluorphosphat wird nach DE 196 411 38 synthetisiert. Das Produkt wird in Acetonitril nach folgender Reaktionsgleichung umgesetzt:

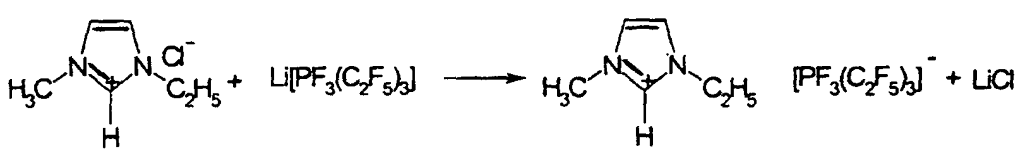

Die Reaktionsmischung wird unter Kühlung über eine Glasfritte vakuumfiltriert, um das als Nebenprodukt gebildete LiCl zu entfernen. Das Lösungsmittel wird unter Vakuum abdestilliert und das erhaltene 1-Ethyl-3-methylimidazolium tris(pentafluorethyl)trifluorphosphat im Vakuum getrocknet.

Beispiel 2

Synthese von 1,2-dimethyl-3-propylimidazolium tris(pentafluorethyl)trifluorphosphat

Lithium tris(pentafluorethyl)trifluorphosphat wird nach DE 196 411 38 synthetisiert. Das Produkt wird in Acetonitril nach folgender Reaktionsgleichung umgesetzt:

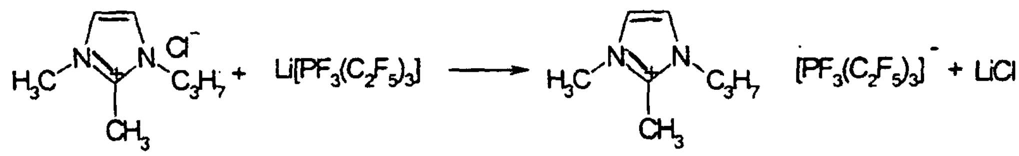

Die Reaktionsmischung wird unter Kühlung über eine Glasfritte vakuumfiltriert, um das als Nebenprodukt gebildete LiCl zu entfernen. Das Lösungsmittel wird unter Vakuum abdestilliert und das erhaltene 1,2-dimethyl-3-propylimidazolium tris(pentafluorethyl)trifluorphosphat im Vakuum getrocknet.

Beispiel 3

Synthese von 1-Ethyl-3-methylimidazolium tris(nonafluorbuthyl)trifluorphosphat

Lithium tris(nonafluorbuthyl)trifluorphosphat wird analog Lithium-tris(pentafluorethyl)trifluorphosphat synthetisiert. Das Produkt wird in Acetonitril nach folgender Reaktionsgleichung umgesetzt:

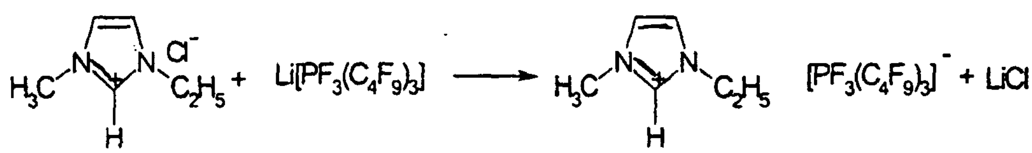

Die Reaktionsmischung wird unter Kühlung über eine Glasfritte vakuumfiltriert, um das als Nebenprodukt gebildete LiCl zu entfernen. Das Lösungsmittel wird unter Vakuum abdestilliert und das erhaltene 1-Ethyl-3-methylimidazolium tris(nonafluorbuthyl)trifluorphosphat im Vakuum getrocknet.

## Patentansprüche

1. Ionische Flüssigkeiten der allgemeinen Formel

$$K^+A^- \qquad (I)$$

worin bedeuten:

$K^+$ ein Kation ausgewählt aus der Gruppe

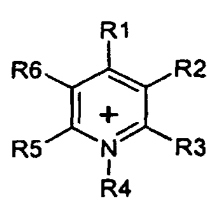

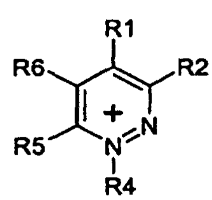

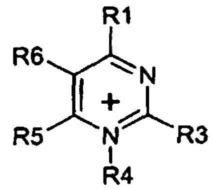

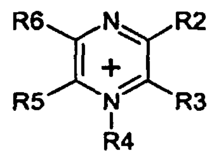

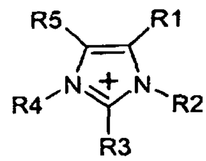

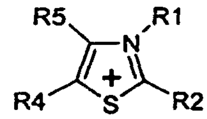

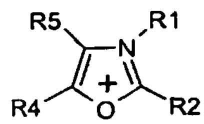

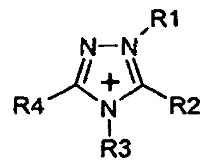

wobei $R^1$ bis $R^5$ gleich oder verschieden, gegebenenfalls durch eine Einfach- oder Doppelbindung direkt miteinander verbunden sind und jeweils einzeln oder gemeinsam folgende Bedeutung haben:

- H,
- Halogen,
- Alkylrest ($C_1$ bis $C_8$) der teilweise oder vollständig durch weitere Gruppen, vorzugsweise F, Cl, N $(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit $1<n<6$ und $0<x\leq 2n+1$ substituiert sein kann

und

$A^-$ Tris(pentafluorethyl)trifluorphosphat oder Tris(nonafluorbutyl)trifluorphosphat bedeutet.

**2.** Verwendung ionischer Flüssigkeiten wie in Anspruch 1 definiert in Mischungen mit aprotischen Lösungsmitteln.

**3.** Verwendung ionischer Flüssigkeiten wie in Anspruch 1 definiert in Mischungen mit anderen Leitsalzen.

**4.** Verwendung ionischerFlüssigkeiten wie in Anspruch 1 definiert in elektrochemischen Zellen.

**5.** Verwendung ionischer Flüssigkeiten wie in Anspruch 1 definiert in Superkondensatoren.

**6.** Verwendung ionischer Flüssigkeiten wie in Anspruch 1 definiert als Lösungsmittel und in der Katalyse von chemischen Reaktionen.

**7.** Verwendung ionischer Flüssigkeiten wie in Anspruch 1 definiert als hydraulische Flüssigkeit.

## Claims

**1.** Ionic liquids of the general formula

$$K^+A^- \qquad (I)$$

in which:

$K^+$ denotes a cation selected from the group

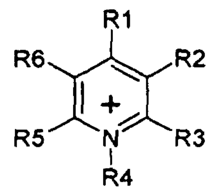 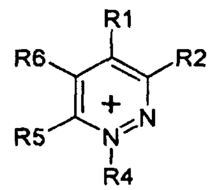

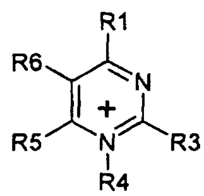 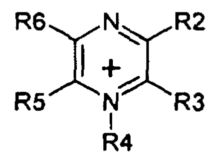

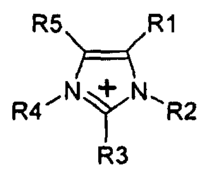 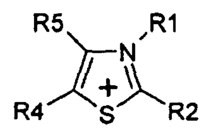

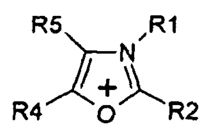 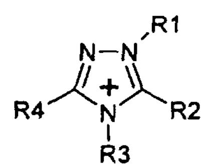

where $R^1$ to $R^5$, identical or differently, are optionally bonded directly to one another by a single or double bond and each, individually or together, have the following meaning:

- H,
- halogen,
- an alkyl radical ($C_1$ to $C_8$), which may be partially or fully substituted by further groups, preferably F, Cl, N ($C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$, where $1<n<6$ and $0<x\leq 2n+1$ and

  $A^-$ denotes tris(pentafluoroethyl)trifluorophosphate or tris(nonafluorobutyl)trifluorophosphate.

**2.** Use of ionic liquids as defined in Claim 1 in mixtures with aprotic solvents.

**3.** Use of ionic liquids as defined in Claim 1 in mixtures with other conductive salts.

**4.** Use of ionic liquids as defined in Claim 1 in electrochemical cells.

5. Use of ionic liquids as defined in Claim 1 in supercapacitors.

6. Use of ionic liquids as defined in Claim 1 as solvents and in the catalysis of chemical reactions.

7. Use of ionic liquids as defined in Claim 1 as hydraulic liquid.

**Revendications**

1. Liquides ioniques de la formule générale

$$K^{+}A^{-} \quad (I)$$

dans laquelle:

$K^+$ représente un cation choisi parmi le groupe

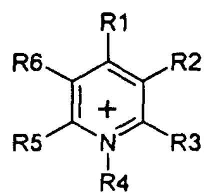
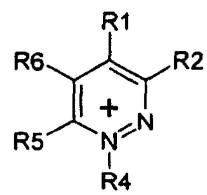
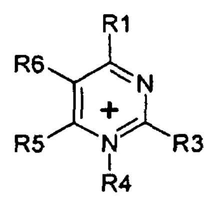
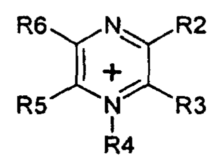
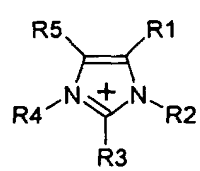
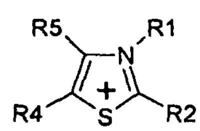
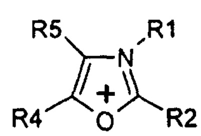
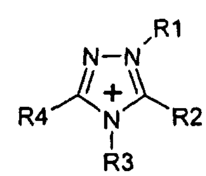

où $R^1$ à $R^5$, identiques ou différents, sont en option liés directement les uns aux autres par une liaison simple ou double et présentent chacun, de façon individuelle ou en association, la signification qui suit:

- H,
- halogène,

- un radical alkyle ($C_1$ à $C_8$), lequel peut être partiellement ou complètement substitué par d'autres groupes, de préférence F, Cl, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$, où $1 < n < 6$ et $0 < x \leq 2n+1$
et

$A^-$ représente tris(pentafluoroéthyl)trifluorophosphate ou tris(nonafluorobutyl)trifluorophosphate.

**2.** Utilisation de liquides ioniques selon la revendication 1 dans des mélanges avec des solvants aprotiques.

**3.** Utilisation de liquides ioniques selon la revendication 1 dans des mélanges avec d'autres sels conducteurs.

**4.** Utilisation de liquides ioniques selon la revendication 1 dans des cellules électrochimiques.

**5.** Utilisation de liquides ioniques selon la revendication 1 dans des supercondensateurs.

**6.** Utilisation de liquides ioniques selon la revendication 1 en tant que solvants et lors de la catalyse de réactions chimiques.

**7.** Utilisation de liquides ioniques selon la revendication 1 en tant que liquide hydraulique.